(19) Europäisches Patentamt
European Patent Office
Office européen des brevets

(11) **EP 4 212 133 A1**

(12) **EUROPEAN PATENT APPLICATION**
published in accordance with Art. 153(4) EPC

(43) Date of publication:
**19.07.2023 Bulletin 2023/29**

(21) Application number: **21866852.3**

(22) Date of filing: **10.09.2021**

(51) International Patent Classification (IPC):
**A61F 2/915** *(2013.01)*

(52) Cooperative Patent Classification (CPC):
**A61F 2/915**

(86) International application number:
**PCT/JP2021/033253**

(87) International publication number:
**WO 2022/054895 (17.03.2022 Gazette 2022/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.09.2020 JP 2020152496**

(71) Applicants:
• **JMR Co., Ltd.**
**Niigata-shi, Niigata, 959-0511 (JP)**

• **Nobelpharma Co., Ltd.**
**Chuo-ku**
**Tokyo 104-0033 (JP)**

(72) Inventors:
• **SASAZAKI, Atsushi**
**Niigata-shi, Niigata 959-0511 (JP)**
• **HANZAWA, Kazuhiko**
**Niigata-shi, Niigata 950-0912 (JP)**

(74) Representative: **Schön, Christoph**
**Dr. Schön, Neymeyr & Partner mbB**
**Bavariaring 26**
**80336 München (DE)**

(54) **STENT AND METHOD FOR PRODUCING STENT**

(57)     A stent having a tubular shape in a self-expanding state, including: a first wire group including a plurality of wires; and a second wire group including a plurality of wires intersecting with the wires of the first wire group at a plurality of locations, wherein the wires of the first wire group and the wires of the second wire group intersect with each other to form a plurality of cells having a substantially rhombic shape in a state of being self-expanding in a tubular shape, and a cross-sectional shape of each of the wires is a rectangular shape thick in a radial direction of the tubular shape.

[FIG.1]

**Description**

TECHNICAL FIELD

The present invention relates to a technical field of a stent, and particularly to a structure of a self-expanding stent suitable for surgery of aortic dissection.

Background Art

Conventionally, for aortic diseases, a thoracotomy surgery has been performed most commonly. The thoracotomy surgery is a surgery of literally performing thoracotomy and replacing a disease site with an artificial blood vessel, but a physical burden on a patient is high, and a death ratio is also high. Even if the surgery is successful, it is a reality that complications and the like are developed and a patient is bedridden in many cases.

Thereafter, it is also a reality that "stent graft surgery" is performed via the aorta by a catheter inserted from a groin without thoracotomy (see Non Patent Literature 1). Since thoracotomy is not required in the "stent graft surgery", there is an advantage that surgery time is short and a physical burden and an economic burden on a patient can be reduced.

Citation List

Patent Literature

Patent Literature 1: JP H11-57018A

Non Patent Literature

Non Patent Literature 1: The Japanese Association for Thoracic Surgery, Website on Thoracic Aortic Aneurysms http://www.jpats.org/modules/general/index.php?content_id=1 6

Summary of Invention

Technical Problem

However, meanwhile, in a case of a disease such as arch aorta dissection, which is common in Japanese, it is extremely difficult to perform a surgery using this stent graft at present because there is a branch blood vessel in the arch aorta. Since the stent graft has a "membrane" structure on a surface of the stent, if the stent graft is directly placed on the arch aorta, a blood flow to the branch blood vessel is inhibited. Therefore, a method for partially opening (fenestration) the membrane only at a position corresponding to the branch blood vessel has also been proposed. However, it is extremely difficult to accurately position the branch blood vessel and the opening when the stent graft having the opening is disposed at a disease site with a catheter, and complications due to inconsistency are often developed.

On the other hand, there is a demand for using a general stent having no membrane structure (see Patent Literature 1), but the general stent has poor flexibility against bending in a direction perpendicular to an axial direction, and is usually not suitable for use in the arch aorta. For example, in a case of an all-linked stent illustrated in Fig. 19, when the stent is bent, a hollow portion is crushed and a blood flow cannot be ensured as illustrated in Fig. 20. In a case of a partially linked stent illustrated in Fig. 21, although the stent exhibits flexibility against bending to a certain degree in a specific direction (see Fig. 22), there is a problem that the stent kinks when being bent at a certain curvature or more (see Fig. 23). In addition, there is a problem in handleability, for example, when the stent is contracted and housed in a catheter, a break of a link is likely to be caught by the catheter, and it is difficult to house the stent in the catheter.

Therefore, the present invention has been made in order to solve these problems, and an object of the present invention is to provide a stent that does not include such a membrane as in a stent graft, has sufficient kink resistance, and has excellent flexibility, and a method for manufacturing the stent.

Solution to Problem

That is, the stent according to the present invention is a stent having a tubular shape in a self-expanding state, the stent including: a first wire group including a plurality of wires ;and a second wire group including a plurality of wires intersecting with the wires of the first wire group at a plurality of locations, in which the wires of the first wire group and the wires of the second wire group intersect with each other to form a plurality of cells having a substantially rhombic shape in a state of being self-expanding in a tubular shape, and a cross-sectional shape of each of the wires is a rectangular shape thick in a radial direction of the tubular shape.

A method for manufacturing a stent according to the present invention is a method for manufacturing a stent, including: a step of forming, from a tubular body made of a shape memory alloy, by laser processing, a mesh tubular body in which meandering structures in which wires meander in a circumferential direction at an equal pitch are arranged in an axial direction, and apexes of arc portions of the adjacent meandering structures are connected to each other to be integrated; a step of enlarging the mesh tubular body in diameter to a predetermined diameter; and a step of causing the mesh tubular body to memorize the shape thereof in a state where the mesh tubular body is enlarged in diameter, in which the wires intersect with each other to form a plurality of cells having a substantially rhombic shape in a state where the mesh tubular body is enlarged in diameter to the predetermined diameter, and a cross-sectional shape of each of the wires is a rectangular shape thick in the radial direction.

Advantageous Effects of Invention

The present invention can provide a stent that does not include such a membrane as in the stent graft, has sufficient kink resistance, and has excellent flexibility, and a method for manufacturing the stent.

Brief Description of Drawings

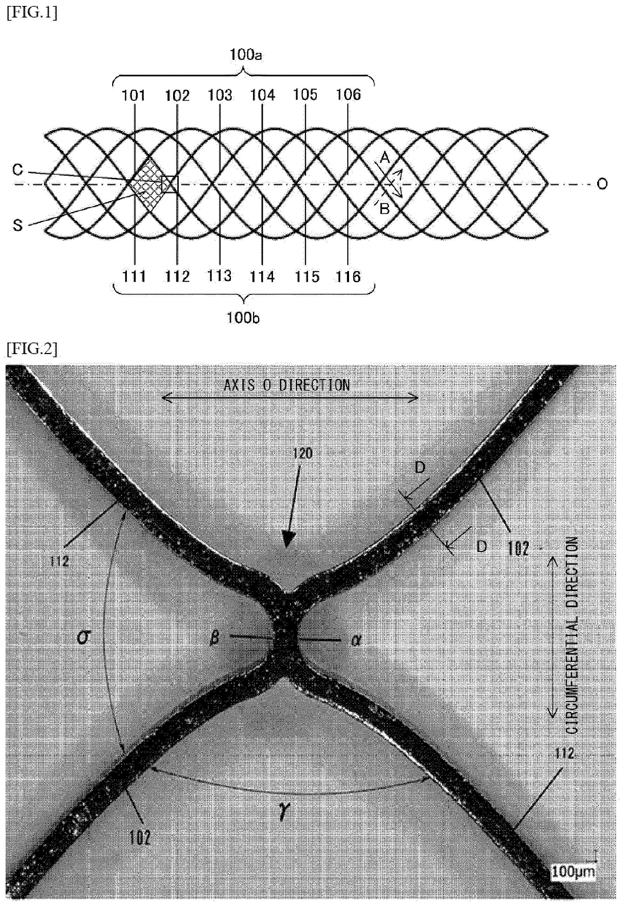

Fig. 1 is a schematic front view of a stent according to an embodiment of the present invention in a self-expanding state.
Fig. 2 is an enlarged photograph illustrating a wire intersecting portion in a range C of Fig. 1.

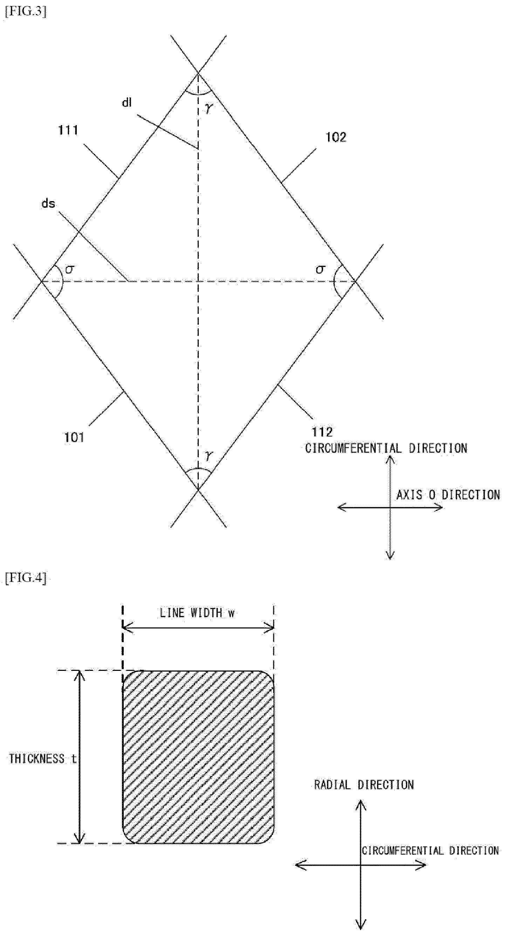

Fig. 3 is an enlarged schematic diagram of a cell S in Fig. 1.
Fig. 4 is a cross-sectional view taken along line D-D in Fig. 2.

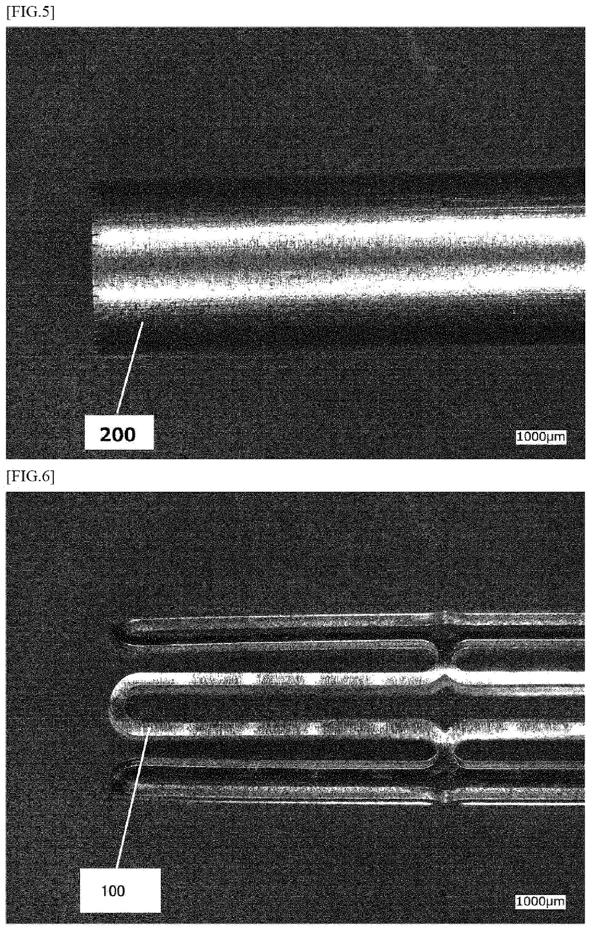

Fig. 5 is a photograph of a tube made of a shape memory alloy before processing.
Fig. 6 is a photograph of a stent made of a shape memory alloy after tube processing.

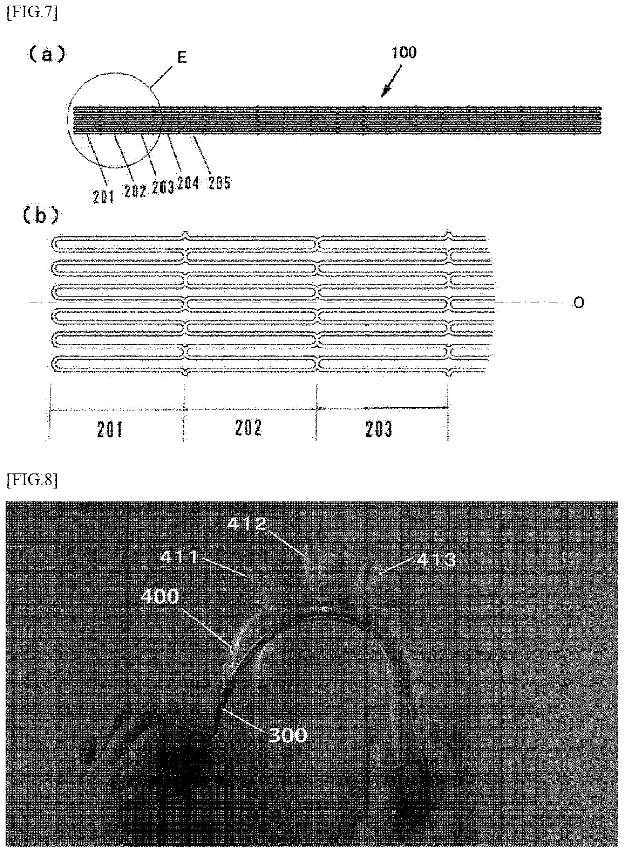

Fig. 7(a) is an overall view of a stent before self-expansion, and Fig. 7(b) is an enlarged view of a range E of Fig. 7(a).
Fig. 8 is a photograph illustrating a state before the stent according to the embodiment of the present invention is exposed from a catheter.

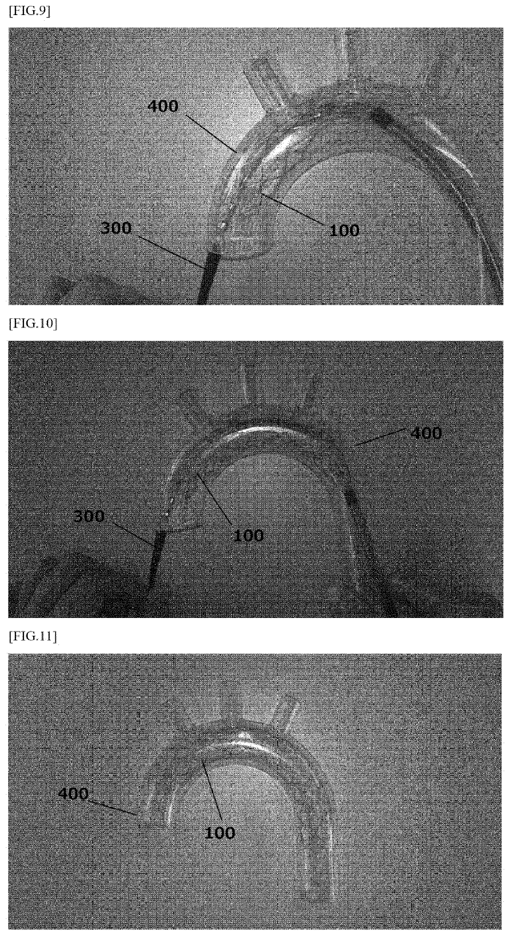

Fig. 9 is a photograph illustrating a state in which the stent according to the embodiment of the present invention is started to be exposed from the catheter.
Fig. 10 is a photograph illustrating a state in which the stent according to the embodiment of the present invention is further exposed from the catheter.
Fig. 11 is a photograph illustrating a state in which placement of the stent according to the embodiment of the present invention is completed.

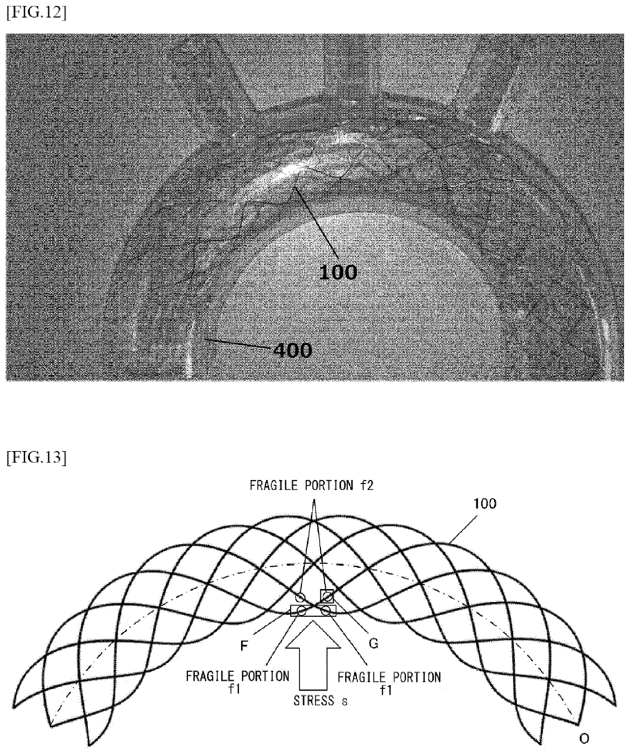

Fig. 12 is a partially enlarged photograph of Fig. 11.
Fig. 13 is a schematic front view when the stent according to the embodiment of the present invention is curved in a self-expanding state.

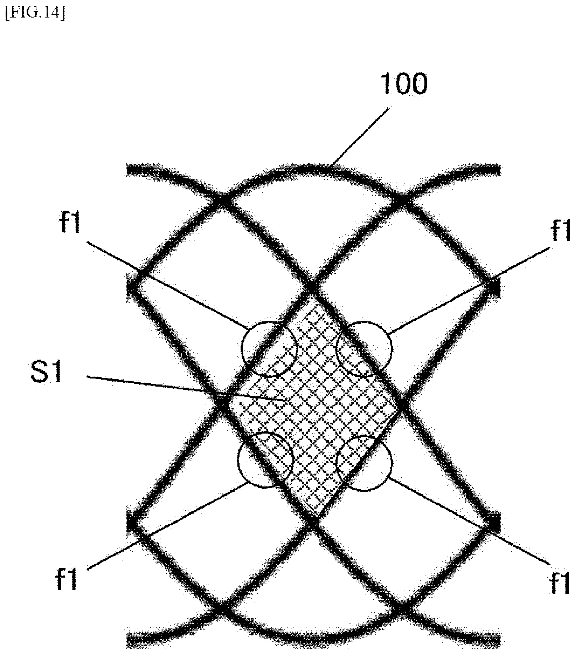

Fig. 14 is a bottom view of a range F of Fig. 13 as viewed from a stress direction.

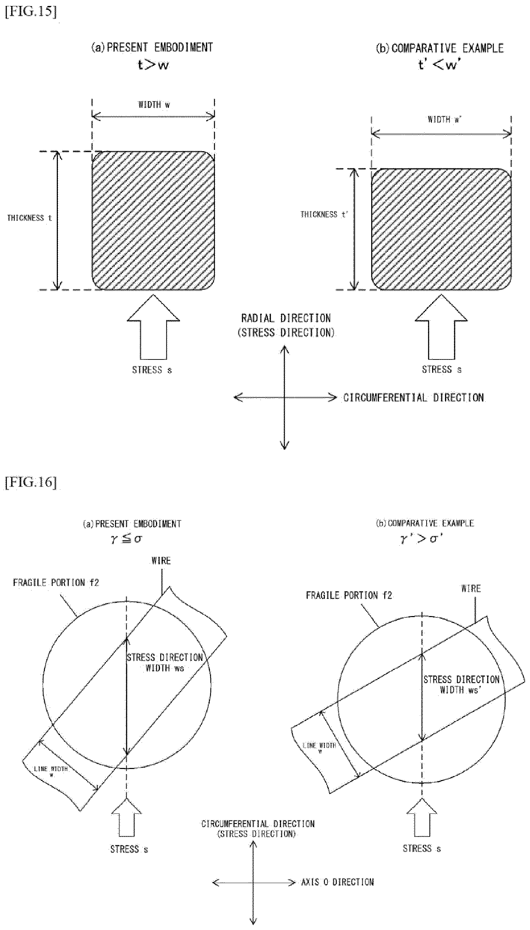

Fig. 15 is a cross-sectional view comparing a wire cross section of the stent according to the embodiment of the present invention with a wire cross section of a comparative example.
Fig. 16 is an enlarged front view comparing the wire in a range G of Fig. 13 with the wire of the comparative example.

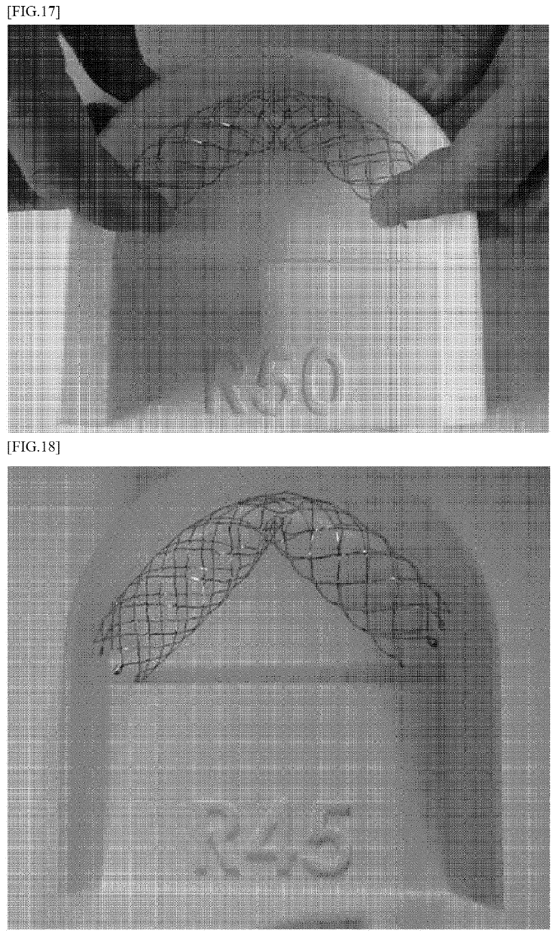

Fig. 17 is a photograph illustrating a state in which a stent of Example 4 is fitted into a mold of R50.
Fig. 18 is a photograph illustrating a state in which the stent of Example 4 is fitted into a mold of R45.

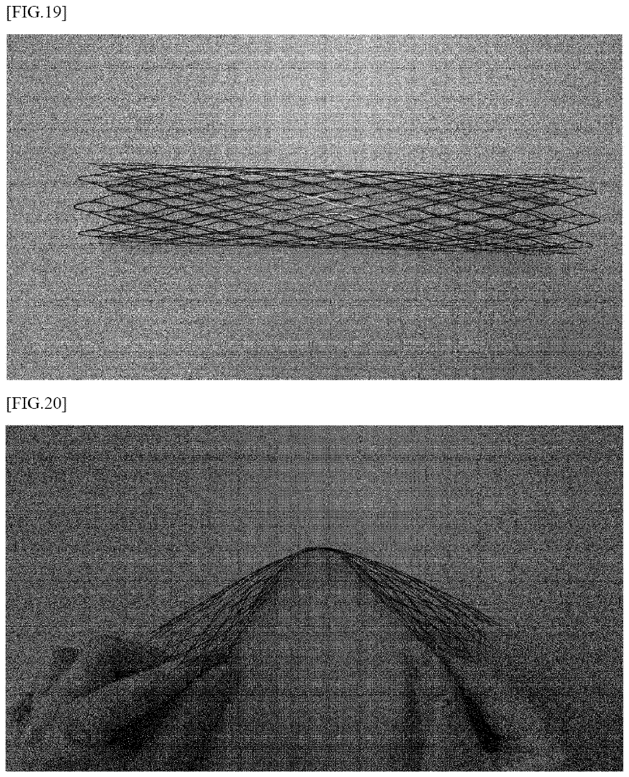

Fig. 19 is an entire photograph of an all-linked stent as a conventional example.
Fig. 20 is a photograph illustrating a state in which the all-linked stent in Fig. 19 is bent.

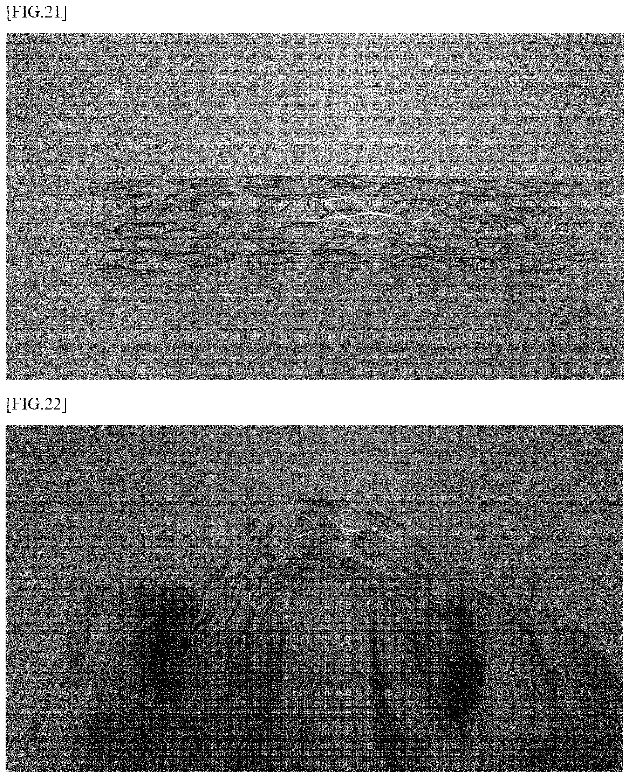

Fig. 21 is an entire photograph of a partially linked stent as a conventional example.
Fig. 22 is a photograph illustrating a state in which the partially linked stent in Fig. 21 is bent in a specific direction.

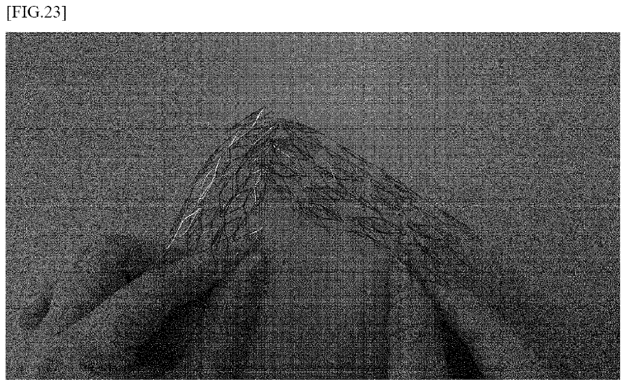

Fig. 23 is a photograph illustrating a state in which the partially linked stent in Fig. 21 is bent in a direction other than the specific direction.

Description of Embodiments

Hereinafter, a stent and a method for manufacturing the stent according to an embodiment of the present invention will be described with reference to the attached drawings. Note that, for easy understanding of the drawings, the size and dimensions of each part are exaggerated in some parts, and do not necessarily coincide with those of an actual product.

(Configuration of Stent)

Fig. 1 illustrates a schematic front view of a stent according to an embodiment of the present invention in a self-expanding state. Fig. 2 illustrates an enlarged photograph illustrating a wire intersecting portion in a range C of Fig. 1. Fig. 3 illustrates an enlarged schematic diagram of a cell S in Fig. 1. Fig. 4 illustrates a cross-sectional view taken along line D-D in Fig. 2. Hereinafter, the configuration of the stent 100 in a self-expanding state will be described based on these drawings. Note that the self-expanding state refers to a state in which a stent expands to a diameter at the time of use set in specifications as a stent.

The stent 100 illustrated in Fig. 1 is made of metal. The metal constituting the stent 100 is, for example, a nickel-titanium alloy, a cobalt alloy, tantalum, or stainless steel, and is preferably a shape memory alloy that can be deformed from a contracted state to a self-expanding state by body temperature or the like.

The stent 100 of the present embodiment has a length and a diameter suitable for use in the arch aorta, for example, has a total length of 80 mm or more or 100 mm or more and 200 mm or less or 250 mm or less, and a diameter of 15 mm or more or 20 mm or more and 30 mm or less or 40 mm or less in a self-expanding state.

In a self-expanding state as illustrated in Fig. 1, the stent 100 has a first wire group 100a including six wires 101, 102, 103, 104, 105, and 106 that are parallel to one direction (solid arrow A in Fig. 1) oblique to an axial direction and extend in a spiral shape coaxially (on an axis O). In addition, the stent 100 has a second wire group 100b including six wires 111, 112, 113, 114, 115, and 116 that are parallel to an oblique direction (dotted arrow B in Fig. 1) symmetrical with the first wire group 100a against the axis O and extend in a spiral shape coaxially with the first wire group 100a (on the axis O).

Specifically, the six wires 101 to 106 of the first wire group 100a have spiral shapes whose phases are shifted by 60° from each other. The six wires 111 to 116 of the second wire group 100b also have spiral shapes whose phases are shifted by 60° from each other. The first wire group 100a and the second wire group 100b have spiral shapes in different directions, and therefore intersect with each other at a plurality of locations, and form a mesh extending in a tubular shape in the axis O direction as a whole. In the stent 100 constituted in this manner, for example, as illustrated by a hatched portion in Fig. 1, the first wire group 100a and the second wire group 100b intersect with each other to form a plurality of cells S having a substantially rhombic shape (including a square shape).

The intersecting portions between the wires 101 to 106 of the first wire group and the wires 111 to 116 of the second wire group are integrally formed without overlapping with each other in a radial direction of the stent 100. For example, as illustrated in Fig. 2 in which a range C of Fig. 1 is enlarged, an intersecting portion 120 has a shape in which apexes of two arc portions $\alpha$ and $\beta$ of the wire 102 of the first wire group 100a and the wire 112 of the second wire group 100b facing each other in the axis O direction are merged to be integrated. The same applies to other intersecting portions.

In addition, in the stent 100 of the present embodiment, a first intersection angle $\gamma$ which is an angle opened in the axis O direction at the intersecting portion 120 in a self-expanding state is equal to or less than a second intersection angle $\sigma$ which is an angle opened in a circumferential direction (that is, an opening degree of each of the arc portions $\alpha$ and $\beta$). A relationship between the first intersection angle $\gamma$ and the second intersection angle $\sigma$ is preferably $\sigma/\gamma \geq$ 1.22, and more preferably $\sigma/\gamma \geq$ 1.36. A basis for these numerical values will be described in Examples described later. Note that an upper limit of $\sigma/\gamma$ is preferably $\sigma/\gamma \leq$ 2.0 (for example, $\gamma$ = 60° and $\sigma$ = 120°) such that the stent 100 can ensure a sufficient length in the axis O direction in a self-expanding state.

That is, such a relationship between the first intersection angle $\gamma$ and the second intersection angle $\sigma$ is such that each cell S formed by the first wire group 100a and the second wire group 100b has a square shape or a rhombus shape long in the circumferential direction in the self-expanding state of the stent 100. When the cell S has a rhombus shape, as illustrated in Fig. 3 obtained by enlarging the cell S portion of Fig. 1, the first intersection angle $\gamma$ is an acute internal angle, and the second intersection angle $\sigma$ is an obtuse internal angle. In addition, a long diagonal line dl of the cell S extends in the circumferential direction of the stent 100, and a short diagonal line ds extends in the axis O direction of the stent 100. That is, when a value of $\sigma/\gamma$ increases, the long diagonal line dl becomes longer, and the shape becomes a rhombus shape longer in the circumferential direction. Note that each apex of the cell S is a central position of the intersecting portion 120, that is, an apex of the two arc portions $\alpha$ and $\beta$.

In addition, as illustrated in Fig. 4 which is a cross-sectional view taken along line D-D in Fig. 2, a cross-sectional shape perpendicular to an extending direction of the wires 101 to 106 of the first wire group 100a and the wires 111 to 116 of the second wire group 100b is a rectangular shape thick in the radial direction of the stent 100.

Specifically, when the thickness of each wire in the radial direction is represented by t and the length of the short width orthogonal to the thickness (that is, line width) is represented by w, a relationship between the thickness t and the line width w is preferably w/t < 0.9. The relationship is more preferably w/t < 0.81. A basis for these numerical values will be described in Examples described later. Note that a lower limit of w/t is preferably w/t $\geq$ 0.5 in consideration of strength and the like at the time of processing for manufacturing the stent 100 and using the stent 100.

As described above, in the stent 100 of the present embodiment, the wires of the first wire group 100a and the second wire group 100b have a rectangular shape thick in the radial direction, and the cell S formed by the first wire group 100a and the second wire group 100b has a square shape or a rhombus shape long in the circumferential direction.

(Method of manufacturing stent)

Fig. 5 illustrates a photograph of a tube made of a shape memory alloy before processing. Fig. 6 illustrates a photograph of a stent made of a shape memory alloy after tube processing. Fig. 7(a) illustrates an overall view of the stent before self-expansion. Fig. 7(b) illustrates an enlarged view of a range E of Fig. 7(a). Hereinafter, a method for manufacturing the stent 100 will be described based on these drawings.

The stent 100 of the embodiment of the present invention is first formed from a tube (tubular body) 200 made of a shape memory alloy as illustrated in Fig. 5. Specifically, laser processing (cutting processing) is performed on the tube 200 so as to cut out an unnecessary portion, and thereafter, surface treatment such as chemical treatment or electrolytic polishing is performed in order to remove burrs and edges, whereby a mesh tubular body as illustrated in Fig. 6 is formed. That is, this mesh tubular body corresponds to the stent 100 in a contracted state. In addition, the stent 100 is enlarged in diameter to a predetermined diameter (for example, a maximum diameter that satisfies the relationship between the first intersection angle $\gamma$ and the second intersection angle $\sigma$ described above), is subjected to heat treatment or the like in a state where the stent 100 is enlarged in diameter to memorize the shape thereof, and is finished as the self-expanding stent 100.

As illustrated in Fig. 7(a), the stent 100 before self-expansion, that is, in a contracted state has a structure in which meandering structures 201, 202, 203, 204, 205 ••• extending in a meandering manner in the circumferential direction at an equal pitch are formed by wires, and the meandering structures 201, 202, 203, 204, 205 ••• are connected to each other in a line in the axis O direction. Specifically, as illustrated in Fig. 7(b), each of the meandering structures 201, 202, 203, 204, 205 ••• is constituted by a linear portion extending in the axis O direction and a semi-circular arc portion folded back in the circumferential direction, and apexes of semi-circular arc portions of adjacent meandering structures are connected to each other to be integrated. In the stent 100 in the contracted state constituted as described above, the diamond-shaped cell S portion in the self-expanding state forms a substantially oval space long in the axis O direction in the contracted state.

(Action and function of stent)

Figs. 8 to 12 are photographs illustrating a state of the stent 100 when the stent 100 of the embodiment of the present invention is placed in an arch aorta model 400 imitating the arch aorta of a human. Hereinafter, an action and a function of the stent 100 will be described using the arch aorta model 400 based on these drawings.

Fig. 8 illustrates a photograph illustrating a state before the stent 100 is exposed from a catheter 300. As illustrated in Fig. 8, similarly to a human body, the arch aorta model 400 has branch blood vessels of a brachiocephalic artery 411, a left common carotid artery 412, and a left subclavian artery 413 on an upper side (head side of the human body). The stent 100 is housed in the catheter 300 in a contracted state. When being actually used in the human body, the catheter 300 is inserted from the femoral aorta or the like through the abdominal aorta and the thoracic aorta to the arch aorta. Then, as illustrated in Fig. 8, a distal end portion of the catheter 300 is curved along the curved shape of the arch aorta model 400, and the stent 100 in a contracted state housed in the catheter 300 also has a shape along the curved shape.

Fig. 9 illustrates a photograph illustrating a state in which the stent 100 starts to be exposed from the catheter 300. Fig. 10 illustrates a photograph illustrating a state in which the stent 100 ends to be exposed from the catheter 300. As illustrated in Fig. 9, the stent 100 is exposed from a distal end side of the catheter 300. The stent 100 self-expands from a portion exposed from the catheter 300 by superelasticity of the shape memory alloy. Then, as illustrated in Fig. 10, when the stent 100 is entirely exposed from the catheter 300, the entire stent 100 is in an expanding state.

Fig. 11 illustrates a photograph illustrating a state in which the stent 100 is completely placed in the arch aorta (model) 400. Fig. 12 illustrates an enlarged view of a main part of Fig. 11. In addition, as illustrated in Figs. 11 and 12, when the entire stent 100 is completely expanded, the catheter 300 is removed from the blood vessel, and only the stent 100 is placed over the entire region of the arch aorta (model) 400.

As described above, in the present embodiment, when the catheter 300 housing the stent 100 is disposed in the arch aorta model 400 and the stent 100 is sequentially delivered, the stent 100 expands (self-expands) to a state in which the shape thereof is memorized by temperature (body temperature in the body). Therefore, the stent 100 sticks to an inner peripheral wall of the arch aorta model 400 so as to sufficiently push and spread the inner peripheral wall and follows the curved shape flexibly.

The kink resistance of the stent 100 of the present embodiment will be described in detail below.

Fig. 13 illustrates a schematic front view when the stent according to the embodiment of the present invention is curved in a self-expanding state. Fig. 14 illustrates a bottom view of a range F of Fig. 13 as viewed from a stress direction (lower side in Fig. 13). Fig. 15 illustrates a cross-sectional view comparing a wire cross section of the present embodiment with a wire cross section of a comparative example. Fig. 16 illustrates an enlarged front view comparing the wire of the present embodiment with the wire of the comparative example in a range G of Fig. 13.

As illustrated in Fig. 13, when the stent 100 of the present embodiment is curved, for example, along the arch aorta, stress concentrates on a central portion in the axis O direction, which is an apex of the curve, that is, the most load is applied to the central portion. That is, a stress s in a direction perpendicular to the axis O direction is received on an inner side of the curve (a center side of a curved arc) of the central portion as indicated by the hollow arrow in Fig. 13. Meanwhile, in the stent 100, a portion of each wire between intersecting portions, surrounded by a circle in Fig. 13 is a fragile portion f1 or f2 that is likely to act as a starting point of kink.

As illustrated in Fig. 14 in which the range F of Fig. 13 corresponding to the stress concentration portion is enlarged, in the stent 100, a cell S1 (hatched portion) corresponding to the stress concentration portion particularly receives a stress. The fragile portion f1 in the cell S1 is a central position of each side of the rhombus.

As illustrated in Fig. 15(a), a cross section perpendicular to the extending direction of the wire corresponding to each fragile portion f1 is thick in the radial direction of the stent 100. Meanwhile, a wire cross section of a comparative example illustrated in Fig. 15(b) has a width w' longer than a thickness t'. Since the stress s acts in the radial direction of the stent 100 in the range F of Fig. 13 corresponding to the stress concentration portion, the wire cross section of the present embodiment having the thickness t in the radial direction has higher strength with respect to the stress s than that in the comparative example and is excellent in kink resistance.

In addition, in the curved stent 100 illustrated in Fig. 13, a stress is relatively concentrated also on a side surface of a central portion in the axis O direction. As illustrated in Fig. 16(a), since a wire extends obliquely with respect to the axis O direction, the stress s acts on the fragile portion f2 in the side surface portion obliquely with respect to the extending direction of the wire.

As described above, in the stent 100 of the present embodiment, the first intersection angle γ which is an angle opened in the axis O direction is equal to or less than the second intersection angle σ which is an angle opened in the circumferential direction. Meanwhile, in a comparative example illustrated in Fig. 16(b), the line width w of a wire is the same as that of the wire of the present embodiment, but the first intersection angle γ' is larger than the second intersection angle σ'.

In this case, a width ws of the wire of the present embodiment (hereinafter, a stress direction width ws) in a stress direction (circumferential direction) in Fig. 16(a) is longer than a stress direction width ws' of the comparative example in Fig. 16(b). That is, the wire of the present embodiment has a more sufficient cross-sectional area in the stress direction. Therefore, even in the side surface portion of the stent 100 in which the stress s acts in the radial direction in the stress concentration portion, a large cross-sectional area in the stress direction can be ensured, the strength against the stress s is higher than that in the comparative example, and the kink resistance is excellent.

As described above, the stent 100 of the present embodiment achieves the flexible stent 100 having excellent kink resistance without including such a membrane as in the stent graft. Absence of the membrane makes it difficult to inhibit a blood flow to a branch blood vessel. In particular, in the stent 100 of the present embodiment, since each wire has a narrow line width w with respect to the thickness t, it is further difficult to inhibit the blood flow. Therefore, it is possible to reduce difficulty for a physician to perform precise stent positioning required to align an opening formed in the stent graft with the branch blood vessel.

Furthermore, since the intersecting portion (for example, 120) is integrally formed without overlapping in the radial direction of the stent 100, the thickness of the intersecting portion (for example, 120) is reduced by half as compared with an overlapping type stent, and unevenness is not generated in the portion. Therefore, insertion into the catheter 300 having a smaller diameter is possible, and the catheter 300 can be operated more smoothly at the time of stent placement with small frictional resistance. Furthermore, a blood vessel wall is less affected (stimulated). In addition, as compared with a wire braided stent, an expansion force of the stent itself can be ensured strongly, and the stent is also resistant to crush due to a pressing force caused by a blood flow that can flow into a detachment side.

Note that, in the stent 100 of the above embodiment, a metal tube is subjected to laser processing to be formed into a shape in which the wires 101 to 106 of the first wire group and the wires 111 to 116 of the second wire group are integrated, but the intersecting portion of the spiral wires may be fixed by heat or the like. In addition, the stent 100 may be made of a spring material. In a state where the stent 100 is enlarged in diameter, annealing is performed such that the spring material is in a free state, and the stent 100 is contracted when the stent is inserted into a catheter. Then, by exposing the stent 100 from the catheter, the stent 100 is enlarged in diameter by a reaction force of the spring. Also in this case, by forming the cross sections of the wires 101 to 106 of the first wire group and the wires 111 to 116 of the second wire group into a rectangular shape thick in the radial direction and setting the first intersection angle γ to be equal to or less than the second intersection angle σ, the first wires 101 to 106 and the second wires 111 to 116 are enlarged in diameter into a tubular shape, kink resistance to curving can be ensured while an internal space of the stent 100 is ensured, and flexibility can be ensured as a whole.

(Examples)

Hereinafter, results of a kink resistance test of the stent according to the present embodiment will be described with reference to Tables 1 to 5 and Figs. 17 and 18.

Table 1 presents design specifications of a base material and a stent in each of Examples. Table 2 presents final specifications of a stent after forming in each of Examples. Table 3 presents results of a kink resistance test in each of Examples.

[Table 1]

| (unit: mm) | Base material | | Specifications of stent | | | | | | |
|---|---|---|---|---|---|---|---|---|---|
| Example | Outer diameter | Thickness | Line width | Link portion | Crown | Cell (M to M) | Slit length | Marker | Total length |
| 1 | 3 | 0.3 | 0.200 | 0.300 | 6 | 11 | 13.34 | Present | 160 |
| 2 | 3 | 0.3 | 0.225 | 0.338 | 6 | 11 | 13.34 | Present | 160 |
| 3 | 3 | 0.3 | 0.250 | 0.375 | 6 | 11 | 13.34 | Present | 160 |
| 4 | 3 | 0.3 | 0.300 | 0.450 | 6 | 11 | 13.34 | Present | 160 |
| 5 | 3 | 0.3 | 0.275 | 0.412 | 6 | 11 | 13.34 | Present | 160 |
| 6 | 3 | 0.3 | 0.245 | 0.368 | 6 | 9 | 16.00 | Present | 154 |
| 7 | 3 | 0.3 | 0.250 | 0.368 | 6 | 9 | 15.00 | Present | 145 |
| 8 | 3 | 0.3 | 0.245 | 0.368 | 6 | 8 | 17.00 | Present | 145 |
| 9 | 3 | 0.3 | 0.245 | 0.368 | 6 | 10 | 14.00 | Present | 150 |
| 10 | 3 | 0.3 | 0.245 | 0.368 | 6 | 8 | 18.00 | Present | 153 |

Specifically, Table 1 presents an outer diameter and a thickness of a base material which is a tube made of a shape memory alloy, and the outer diameter and the thickness of the base material in each of Examples are 3 mm and 0.3 mm, respectively. In addition, as design specifications of a stent, a line width, a width of a link portion (a length of a wire intersecting portion in an axial direction), the number of crowns, the number of cells, a slit length (a length of a substantially oval space in an axis O direction), presence or absence of a marker, and a total length (a length in the axis O direction) are described. Note that the marker is formed by welding a material having high radiation opacity, such as tantalum, to both ends of a stent in order to enhance visibility of the stent at the time of X-ray photography. The number of cells is the number of cells between markers (M to M) at both ends of a stent.

[Table 2]

| | | Final specifications | | | | | | | | | | | |
|---|---|---|---|---|---|---|---|---|---|---|---|---|---|
| (unit: mm) | | | | | | | | | | | | | |
| Example | Maximum diameter | Total length | Shortening ratio | Polishing ratio | Thickness t | Line width w | Wire cross-sectional area [mm2] | Cell width | Cell height | Cell area [mm2] | γ (degrees) | σ (degrees) | 80% [N/cm] |
| 1 | 20 | 114 | 71% | 18% | 0.257 | 0.160 | 0.041 | 9.83 | 10.23 | 109.56 | 91 | 91 | 2.1 |
| 2 | 20 | 113 | 71% | 18% | 0.240 | 0.202 | 0.048 | 9.56 | 10.38 | 134.29 | 81 | 94 | 2.5 |
| 3 | 20 | 113 | 71% | 18% | 0.245 | 0.209 | 0.051 | 9.53 | 10.35 | 118.84 | 82 | 95 | 3.8 |
| 4 | 20 | 112 | 70% | 18% | 0.247 | 0.240 | 0.059 | 9.46 | 10.4 | '98.38 | 80 | 97 | 5.3 |
| 5 | 20 | 114 | 71% | 18% | 0.237 | 0.228 | 0.054 | 9.65 | 10.4 | 100.36 | 83 | 96 | 4.4 |
| 6 | 24 | 108 | 70% | 18% | 0.240 | 0.202 | 0.048 | 10.98 | 12.23 | 134.29 | 79 | 96 | 2.1 |
| 7 | 24 | 92 | 63% | 18% | 0.245 | 0.209 | 0.051 | 9.13 | 12.25 | 111.84 | 78 | 106 | 3.2 |
| 3 | 24 | 107 | 74% | 18% | 0.240 | 0.209 | 0.050 | 12.52 | 12.77 | 159.88 | 91 | 89 | 2.1 |
| 9 | 24 | 83 | 55% | 18% | 0.249 | 0.202 | 0.050 | 7.84 | 12.27 | 96.20 | 66 | 113 | 4.6 |
| 10 | 24 | 119 | 78% | 18% | 0.241 | 0.216 | 0.052 | 13.64 | 12.71 | 173.36 | 96 | 83 | 1.9 |

Table 2 presents specifications of a stent in a self-expanding state immediately before the kink resistance test is performed, and describes an expanded maximum diameter, a total length (a length in the axis O direction), a shortening ratio (total length in a self-expanding state/total length in a contracted state), a polishing ratio by electrolytic polishing, a thickness t, a line width w, a wire cross-sectional area, a cell width (short diagonal line of a cell), a cell height (long diagonal line of a cell), a cell area, a first intersection angle $\gamma$, a second intersection angle $\sigma$, and 80% [N/cm] (a pressure value per cm when a stent is contracted to 80% with a maximum expansion state as 100%) in each of Examples.

[Table 3]

| Example | Results of kink resistance test | | | | | | | |
|---|---|---|---|---|---|---|---|---|
| | R75 | R70 | R65 | R60 | R55 | R50 | R45 | R40 |
| 1 | ○ | ○ | ○ | ○ | ○ | ○ | ○ | ○ |
| 2 | ○ | ○ | ○ | ○ | ○ | ○ | ○ | × |
| 3 | ○ | ○ | ○ | ○ | ○ | ○ | ○ | × |
| 4 | ○ | ○ | ○ | ○ | ○ | ○ | × | |
| 5 | ○ | ○ | ○ | ○ | ○ | ○ | ○ | × |
| 6 | ○ | ○ | ○ | ○ | ○ | ○ | ○ | ○ |
| 7 | ○ | ○ | ○ | ○ | ○ | ○ | ○ | ○ |
| 8 | ○ | ○ | ○ | ○ | ○ | ○ | ○ | ○ |
| 9 | ○ | ○ | ○ | ○ | ○ | ○ | ○ | ○ |
| 10 | ○ | ○ | ○ | ○ | ○ | ○ | × | |

Table 3 presents results of the kink resistance test, and presents whether or not kink occurred when stents in Examples were fitted into molds having different curvatures. A case where no kink occurred is indicated by ○, and a case where kink occurred is indicated by ×.

For example, Fig. 17 illustrates a state in which a stent of Example 4 is fitted into a mold of R50 (curvature radius: 50 mm), and when the stent is curved with a smooth curve as illustrated in Fig. 17, it is determined that no kink has occurred. Meanwhile, Fig. 18 illustrates a state in which the stent of Example 4 is fitted into a mold of R45 (curvature radius: 45 mm), and when bending occurs at a central portion of the stent in the axis O direction as illustrated in Fig. 18, it is determined that kink has occurred.

Table 3 indicates that the stents in Examples 1 and 6 to 9 have the best kink resistance, the stents in Examples 2, 3, and 5 have the second-best kink resistance, and the stents in Examples 4 and 10 have poor kink resistance. Since the curvature of the arch aorta is usually R45 or more, Examples other than Examples 4 and 10 are suitable for use in the arch aorta.

[Table 4]

| (unit: mm) | | | | | | | | | | | |
|---|---|---|---|---|---|---|---|---|---|---|---|
| Example | Final specifications | | | Results of kink resistance test | | | | | | | |
| | Thickness t | Line width w | w/t | R75 | R70 | R65 | R60 | R55 | R50 | R45 | R40 |
| 4 | 0.247 | 0.240 | 0.97 | ○ | ○ | ○ | ○ | ○ | ○ | × | |
| 5 | 0.237 | 0.228 | 0.96 | ○ | ○ | ○ | ○ | ○ | ○ | ○ | × |
| 10 | 0.241 | 0.216 | 0.90 | ○ | ○ | ○ | ○ | ○ | ○ | × | |
| 8 | 0.240 | 0.209 | 0.87 | ○ | ○ | ○ | ○ | ○ | ○ | ○ | ○ |
| 3 | 0.245 | 0.209 | 0.85 | ○ | ○ | ○ | ○ | ○ | ○ | ○ | × |
| 7 | 0.245 | 0.209 | 0.85 | ○ | ○ | ○ | ○ | ○ | ○ | ○ | ○ |
| 2 | 0.240 | 0.202 | 0.84 | ○ | ○ | ○ | ○ | ○ | ○ | ○ | × |
| 6 | 0.240 | 0.202 | 0.84 | ○ | ○ | ○ | ○ | ○ | ○ | ○ | ○ |

(continued)

| (unit: mm) | | | | | | | | | | | |
|---|---|---|---|---|---|---|---|---|---|---|---|
| Example | Final specifications | | | Results of kink resistance test | | | | | | | |
| | Thickness t | Line width w | w/t | R75 | R70 | R65 | R60 | R55 | R50 | R45 | R40 |
| 9 | 0.249 | 0.202 | 0.81 | ○ | ○ | ○ | ○ | ○ | ○ | ○ | ○ |
| 1 | 0.257 | 0.160 | 0.62 | ○ | ○ | ○ | ○ | ○ | ○ | ○ | ○ |

Table 4 presents results of calculating w/t of each of Examples based on the thickness t and the line width w in Table 2, and sorting w/t in descending order together with the results of the kink resistance test in Table 3. As presented in Table 4, it can be seen that when w/t is 0.87 or less, kink resistance up to R45 is exhibited, and when w/t is 0.81 or less, kink resistance up to R40 is reliably exhibited. Therefore, in a relationship between the wire thickness t and the line width w in a stent, w/t < 0.90 is preferable, and w/t ≤ 0.81 is more preferable.

[Table 5]

| (unit: mm) | | | | | | | | | | | |
|---|---|---|---|---|---|---|---|---|---|---|---|
| Example | Final specifications | | | Results of kink resistance test | | | | | | | |
| | γ (degrees) | σ (degrees) | σ/γ | R75 | R70 | R65 | R60 | R55 | R50 | R45 | R40 |
| 10 | 96 | 83 | 0.86 | ○ | ○ | ○ | ○ | ○ | ○ | × | |
| 8 | 91 | 89 | 0.98 | ○ | ○ | ○ | ○ | ○ | ○ | ○ | ○ |
| 1 | 91 | 91 | 1.00 | ○ | ○ | ○ | ○ | ○ | ○ | ○ | ○ |
| 5 | 83 | 96 | 1.16 | ○ | ○ | ○ | ○ | ○ | ○ | ○ | × |
| 3 | 82 | 95 | 1.16 | ○ | ○ | ○ | ○ | ○ | ○ | ○ | × |
| 2 | 81 | 94 | 1.16 | ○ | ○ | ○ | ○ | ○ | ○ | ○ | × |
| 4 | 80 | 97 | 1.21 | ○ | ○ | ○ | ○ | ○ | ○ | × | |
| 6 | 79 | 96 | 1.22 | ○ | ○ | ○ | ○ | ○ | ○ | ○ | ○ |
| 7 | 78 | 106 | 1.36 | ○ | ○ | ○ | ○ | ○ | ○ | ○ | ○ |
| 9 | 66 | 113 | 1.71 | ○ | ○ | ○ | ○ | ○ | ○ | ○ | ○ |

Table 5 presents results of calculating σ/γ of each of Examples based on the first intersection angle γ and the second intersection angle σ in Table 2, and sorting σ/γ in ascending order together with the results of the kink resistance test in Table 3. As presented in Table 5, when σ/γ is 1.22 or more, kink resistance up to R40 is exhibited. Note that, in Example 4 in which σ/γ is 1.21, kink occurs at R45, and therefore σ/γ is preferably 1.22 or more such that kink resistance is more reliably exhibited. Therefore, in a relationship between the first intersection angle γ and the second intersection angle σ in a stent, σ/γ ≥ 1.22 is preferable, and σ/γ ≥ 1.36 is more preferable.

The description of the embodiment and Examples of the present invention is completed here, but aspects of the present invention are not limited to the embodiment and Examples.

Reference Signs List

| 100 | Stent |
|---|---|
| 100a | First wire group |
| 100b | Second wire group |
| 101 to 106, 111 to 116 | Wire |
| 120 | Intersecting portion |
| 200 | Tube (tubular body) |
| 201 to 205 | Meandering structure |

| 300 | Catheter |
|-----|----------|
| 400 | Arch aorta model |
| 411 | Brachiocephalic artery |
| 412 | Left common carotid artery |
| 413 | Left subclavian artery |

**Claims**

1. A stent having a tubular shape in a self-expanding state, comprising:

   a first wire group comprising a plurality of wires; and
   a second wire group comprising a plurality of wires intersecting with the wires of the first wire group at a plurality of locations, wherein
   the wires of the first wire group and the wires of the second wire group intersect with each other so as to form a plurality of cells having a substantially rhombic shape in the self-expanding state in a tubular shape, and
   a cross-sectional shape of each of the wires in the first wire group and the second wire group is a rectangular shape that is thicker in a radial direction of the tubular shape than in a length direction thereof.

2. The stent according to claim 1, wherein

   when a thickness of the cross-sectional shape of each of the wires of the first wire group and the second wire group in the radial direction is represented by t, and a length of a short width orthogonal to the thickness is represented by w, a following formula:
   $w/t < 0.90$ is satisfied.

3. The stent according to claim 1, wherein

   when a thickness of the cross-sectional shape of each of the wires of the first wire group and the second wire group in the radial direction is represented by a, and a length of a short width orthogonal to the thickness is represented by b, a following formula:
   $w/t \leq 0.81$ is satisfied.

4. The stent according to claim 1,
   wherein the rhombic shape of the plurality of the [[cell]]cells in the self-expanding state in a tubular shape [[has]]is a rhombus shape in which a long diagonal line extends in a circumferential direction.

5. The stent according to claim 4, wherein
   when an obtuse angle is represented by $\sigma$ and an acute angle is represented by $\gamma$ in an internal angle, the plurality of the [[cell]] cells in the self-expanding state in a tubular shape satisfy a following formula:

$$\sigma / \gamma \geq 1.22.$$

6. The stent according to claim 4, wherein
   when an obtuse angle is represented by $\sigma$ and an acute angle is represented by $\gamma$ in an internal angle, the plurality of the [[cell]] cells in the self-expanding in a tubular shape satisfies $\sigma/\gamma \geq 1.36$.

7. The stent according to claims claim 1,
   wherein intersecting portions between the wires of the first wire group and the wires of the second wire group are integrally formed without overlapping with each other in the radial direction of the tubular shape.

8. The stent according to claim 1,
   wherein a stent graft is not integrated with the stent.

9. A method for manufacturing a stent, comprising:

   forming[[,]] a mesh tubular body from a tubular body made of a shape memory alloy by laser processing,

wherein in the mesh tubular body, a plurality of meandering structures are arranged in an axial direction of the mesh tubular body, and apexes of arc portions of the meandering structures adjacent to each other are connected to each other and integrated, and

wherein in each of the plurality of the meandering structures, wires meander in a circumferential direction of the mesh tubular body at an equal pitch;

enlarging the mesh tubular body in a diameter direction of the mesh tubular body so as to have a decided diameter; and

causing the mesh tubular body to memorize [[a]]the resulting enlarged shape in the diameter direction of the mesh tubular body,

wherein the wires intersect with each other so as to form a plurality of cells having a substantially rhombic shape in a state where the mesh tubular body is enlarged in the diameter direction to the decided diameter, and a cross-sectional shape of each of the wires [[has]]is a rectangular shape that is thicker [[thick]] in the radial direction of the mesh tubular body than a length direction thereof.

[FIG.1]

[FIG.2]

[FIG.3]

111

102

101

112

CIRCUMFERENTIAL DIRECTION

AXIS O DIRECTION

[FIG.4]

LINE WIDTH w

THICKNESS t

RADIAL DIRECTION

CIRCUMFERENTIAL DIRECTION

14

[FIG.5]

[FIG.6]

[FIG.7]

（a）

E

100

201 202 203 204 205

（b）

O

201   202   203

[FIG.8]

412

411

413

400

300

[FIG.9]

[FIG.10]

[FIG.11]

[FIG.12]

[FIG.13]

[FIG.14]

[FIG.15]

(a) PRESENT EMBODIMENT
t>w

(b) COMPARATIVE EXAMPLE
t'<w'

WIDTH w

THICKNESS t

STRESS s

WIDTH w'

THICKNESS t'

STRESS s

RADIAL DIRECTION
(STRESS DIRECTION)

CIRCUMFERENTIAL DIRECTION

[FIG.16]

(a) PRESENT EMBODIMENT
$\gamma \leqq \sigma$

(b) COMPARATIVE EXAMPLE
$\gamma' > \sigma'$

WIRE

FRAGILE PORTION f2

STRESS DIRECTION
WIDTH ws

LINE WIDTH w

STRESS s

WIRE

FRAGILE PORTION f2

STRESS DIRECTION
WIDTH ws'

LINE WIDTH w

STRESS s

CIRCUMFERENTIAL DIRECTION
(STRESS DIRECTION)

AXIS O DIRECTION

[FIG.17]

[FIG.18]

[FIG.19]

[FIG.20]

[FIG.21]

[FIG.22]

[FIG.23]

<div style="text-align:center">**INTERNATIONAL SEARCH REPORT**</div>

| International application No. |
|---|
| **PCT/JP2021/033253** |

**A.    CLASSIFICATION OF SUBJECT MATTER**

*A61F 2/915*(2013.01)i
FI:   A61F2/915

According to International Patent Classification (IPC) or to both national classification and IPC

**B.    FIELDS SEARCHED**

Minimum documentation searched (classification system followed by classification symbols)

A61F2/915

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 2012-510352 A (MINDFRAME, INC) 10 May 2012 (2012-05-10)   paragraphs [0113]-[0114], [0160], [0174]-[0176], fig. 8A-8C, 24A-24B, 29A-29C | 1-3, 9 |
| Y | paragraphs [0113]-[0114], [0160], [0174]-[0176], fig. 8A-8C, 24A-24B, 29A-29C | 4–8 |
| X | US 2010/0256737 A1 (ABBOTT VASCULAR SOLUTIONS INC.) 07 October 2010 (2010-10-07)   paragraphs [0062]-[0063], [0074], [0095], fig. 1-4, 10-11 | 1-3, 9 |
| Y | paragraphs [0062]-[0063], [0074], [0095], fig. 1-4, 10-11 | 4–8 |
| X | JP 2012-532687 A (CONCENTRIC MEDICAL, INC) 20 December 2012 (2012-12-20)   paragraphs [0016], [0023]-[0024], fig. 1A-1B | 1-3, 9 |
| Y | paragraphs [0016], [0023]-[0024], fig. 1A-1B | 4–8 |
| X | JP 2000-502936 A (ENDOVASCULAR TECHNOLOGIES, INC) 14 March 2000 (2000-03-14)   page 10, line 5 to page 12, line 7, fig. 1-12 | 1-3 |
| Y | page 10, line 5 to page 12, line 7, fig. 1-12 | 4–8 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **07 October 2021** | **02 November 2021** |

| Name and mailing address of the ISA/JP | Authorized officer |
|---|---|
| **Japan Patent Office (ISA/JP)**  **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2021/033253** |

**C.    DOCUMENTS CONSIDERED TO BE RELEVANT**

| Category* | Citation of document, with indication, where appropriate, of the relevant passages | Relevant to claim No. |
| --- | --- | --- |
| Y | US 2010/0145433 A1 (ABBOTT CARDIOVASCULAR SYSTEMS, INC.) 10 June 2010 (2010-06-10)<br>    paragraphs [0061]-[0062], fig. 3A-3B | 4-8 |
| Y | EP 2823793 A1 (ACANDIS GMBH & CO. KG) 14 January 2015 (2015-01-14)<br>    paragraph [0046], fig. 6 | 4-8 |
| Y | JP 2019-517300 A (FEMTOS GMBH) 24 June 2019 (2019-06-24)<br>    paragraph [0051], fig. 5 | 4-8 |
| A | CN 106726038 A (SECOND MILITARY MEDICAL UNIVERSITY, PLA) 31 May 2017 (2017-05-31)<br>    entire text, all drawings | 1-9 |

Form PCT/ISA/210 (second sheet) (January 2015)

# EP 4 212 133 A1

INTERNATIONAL SEARCH REPORT
Information on patent family members

International application No.

**PCT/JP2021/033253**

| Patent document cited in search report | | | Publication date (day/month/year) | Patent family member(s) | | | Publication date (day/month/year) |
|---|---|---|---|---|---|---|---|
| JP | 2012-510352 | A | 10 May 2012 | WO | 2011/082319 | A1 | |
| | | | | paragraphs [0184]-[0185], [0224], [0238]-[0240], fig. 8A-8C, 24A-24B, 29A-29C | | | |
| | | | | US | 2010/0174309 | A1 | |
| | | | | US | 2011/0160763 | A1 | |
| | | | | US | 2010/0256600 | A1 | |
| | | | | US | 2010/0217187 | A1 | |
| US | 2010/0256737 | A1 | 07 October 2010 | US | 2002/0049490 | A1 | |
| | | | | paragraphs [0050]-[0051], [0062], fig. 1-4, 10-11 | | | |
| JP | 2012-532687 | A | 20 December 2012 | WO | 2011/006013 | A1 | |
| | | | | paragraphs [0016], [0023]-[0024], fig. 1A-1B | | | |
| | | | | US | 2011/0009875 | A1 | |
| | | | | US | 2011/0009941 | A1 | |
| | | | | US | 2011/0009940 | A1 | |
| JP | 2000-502936 | A | 14 March 2000 | WO | 1997/025000 | A1 | |
| | | | | page 8, line 7 to page 10, line 29, fig. 1-12 | | | |
| | | | | US | 5993482 | A | |
| | | | | US | 6454795 | B1 | |
| | | | | US | 6719782 | B1 | |
| | | | | US | 2005/0015137 | A1 | |
| US | 2010/0145433 | A1 | 10 June 2010 | (Family: none) | | | |
| EP | 2823793 | A1 | 14 January 2015 | DE | 102013107258 | A1 | |
| | | | | paragraph [0046], fig. 6 | | | |
| JP | 2019-517300 | A | 24 June 2019 | WO | 2017/207689 | A1 | |
| | | | | page 13, lines 8-19, fig. 5 | | | |
| CN | 106726038 | A | 31 May 2017 | (Family: none) | | | |

Form PCT/ISA/210 (patent family annex) (January 2015)

27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP H1157018 A

**Non-patent literature cited in the description**

• Website on Thoracic Aortic Aneurysms. The Japanese Association for Thoracic Surgery